(19) Europäisches Patentamt
European Patent Office
Office européen des brevets

(11) Numéro de publication : **0 463 922 A1**

(12) # DEMANDE DE BREVET EUROPEEN

(21) Numéro de dépôt : **91401606.8**

(22) Date de dépôt : **17.06.91**

(51) Int. Cl.[5] : **C07C 67/11,** C07C 69/63, C07D 213/803, C07C 69/56, C07C 69/68, C07C 69/533

(30) Priorité : **22.06.90 FR 9007817**

(43) Date de publication de la demande : **02.01.92 Bulletin 92/01**

(84) Etats contractants désignés : **CH DE ES FR GB IT LI NL**

(71) Demandeur : **RHONE-POULENC CHIMIE
25, quai Paul Doumer
F-92408 Courbevoie Cédex (FR)**

(72) Inventeur : **Desmurs, Jean-Roger, La Jonquière
Route de Ternay, Communay
F-69360 St Symphorien d'Ozon (FR)**
Inventeur : **Ratton, Serge
13, rue d'Ayen
F-78100 St Germain en Laye (FR)**

(74) Mandataire : **Dutruc-Rosset, Marie-Claude et al
RHONE-POULENC CHIMIE Service Brevets Chimie 25, Quai Paul Doumer
F-92408 Courbevoie Cédex (FR)**

(54) **Procédé d'estérification des acides carboxyliques.**

(57) La présente invention a pour objet un procédé d'estérification des acides carboxyliques. L'invention concerne plus particulièrement un procédé de préparation des esters à partir d'acides carboxyliques difficiles à estérifier.

Le procédé de l'invention est caractérisé par le fait que l'on fait réagir ledit acide avec un dérivé halogéné d'un hydrocarbure aliphatique, cycloaliphatique, cyclo- ou arylaliphatique, en phase liquide essentiellement homogène, en présence d'une amine tertiaire non quaternisable.

EP 0 463 922 A1

La présente invention a pour objet un procédé d'estérification des acides carboxyliques. L'invention concerne plus particulièrement un procédé de préparation des esters à partir d'acides carboxyliques difficiles à estérifier.

Un procédé classique d'estérification directe d'un acide carboxylique consiste à faire réagir celui-ci, avec un alcool, en présence d'un catalyseur acide, notamment l'acide sulfurique.
En raison des conditions de température réactionnelle et d'acidité de milieu, un tel procédé n'est pas approprié pour réaliser l'estérification des alcools tertiaires, voire même secondaires, car les risques de déshydratation sont grands [KIRK-OTHMER - Enyclopedia of Chemical Technology, 3e édition, John Wiley and Sons 9 p. 298 et suivantes]. Dans d'autres cas, certains acides forts comme l'acide trifluoroacétique présentent une réactivité faible et sont donc difficile à estérifier par cette méthode.
De plus, lorsque le groupe COOH est porté par un hétérocycle azoté, la quantité d'acide mise en oeuvre n'est plus catalytique, mais devient stoechiométrique en raison de la neutralisation de la fonction azotée. Il s'ensuit un surcoût dû à la consommation de ce réactif.

Un autre mode d'estérification est de faire réagir l'acide carboxylique avec un dérivé halogéné, notamment un halogénure d'alkyle, en milieu biphasique et en présence d'une base forte.
Un des inconvénients de ce procédé est qu'il se produit dans les conditions de la réaction, une hydrolyse de l'halogénure mis en oeuvre, ce qui conduit à la formation d'un alcool dans la phase aqueuse qu'il est ensuite nécessaire d'éliminer. Il y a donc perte de rendement par rapport à la quantité d'halogénure engagé.
En outre, un tel procédé est difficilement applicable à l'estérification de certains acides carboxyliques par exemple, les acides acétoxybenzoïques qui risquent de s'hydrolyser en milieu basique en acides hydroxybenzoïques ou les acides porteurs de groupes fonctionnels susceptibles de réagir, par exemple, les acides aminés ayant une fonction amine protégée.

Un des objectifs de la présente invention est de fournir un procédé d'estérification des acides carboxyliques qui permet d'éviter les inconvénients précités.

Un autre objectif de l'invention est de disposer d'un procédé qui s'applique à une large gamme d'acides carboxyliques.

La présente invention a pour objet un procédé d'estérification d'un acide carboxylique aliphatique, carbocyclique ou hétérocyclique, cyclo-ou arylaliphatique caractérisé par le fait qu'il consiste à faire réagir ledit acide avec un dérivé halogéné d'un hydrocarbure aliphatique, cycloaliphatique, cyclo- ou arylaliphatique, en phase liquide essentiellement homogène, en présence d'une amine tertiaire non quaternisable.

On utilisera ci-après le terme générique de "dérivé halogéné" pour désigner les composés halogénés précités.

Par l'expression "amine non quaternisable", on entend une amine qui, placée dans les conditions de la réaction, mais en l'absence du substrat "acide carboxylique", n'est pas quaternisée par le dérivé halogéné ou ne l'est qu'à un faible taux correspondant à un taux de transformation du dérivé halogéné en amine quaternisée inférieur à 10 %, de préférence inférieure ou égal à 5 %.

Conformément au procédé de l'invention, on fait réagir l'acide carboxylique et le dérivé halogéné en phase liquide essentiellement homogène. On entend par cette expression que les différents réactifs sont liquides dans les conditions réactionnelles mais le procédé n'exclut pas la présence d'éventuels catalyseurs sous forme solide.

La réaction d'estérification selon l'invention peut être conduite en l'absence de solvant ou en présence de solvant organique. D'une manière préférentielle, on préfère choisir les conditions qui permettent de travailler en milieu homogène.

Le procédé de l'invention s'applique à tout acide carboxylique mono- ou polycarboxylique tel que les acides aliphatiques saturés ou insaturés ; carbocycliques ou hétérocycliques, saturés, insaturés ou aromatiques, monocycliques ou polycycliques ; cyclo- ou arylaliphatiques saturés ou insaturés.

Ledit acide peut être symbolisé par la formule (I) suivante:

$$R\text{-}COOH \quad (I)$$

dans laquelle R représente un radical hydrocarboné, éventuellement substitué comportant de 1 à 40 atomes de carbone.

L'invention s'applique plus particulièrement aux composés de formule (I) dans laquelle le radical R symbolise :

1° - un radical aliphatique acyclique, saturé ou insaturé, linéaire ou ramifié,

2° - un radical carbocyclique, saturé, insaturé ou aromatique, monocyclique ou polycyclique.

Ces radicaux peuvent former entre eux des systèmes orthocondensés ou ortho et péricondensés.

3° - un radical hétérocyclique, saturé, insaturé ou aromatique, monocyclique ou polycyclique.

Par "radical hétérocyclique polycyclique, on définit :

– un radical constitué par au moins 2 hétérocycles aromatiques ou non, contenant au moins un hétéroatome dans chaque cycle et formant entre eux des systèmes ortho ou ortho et péricondensés,

– un radical constitué par au moins un un cycle hydrocarboné aromatique ou non et au moins un hétérocycle aromatique ou non, formant entre eux des systèmes ortho ou ortho et péricondensés.

4° - un radical monovalent constitué par un enchaînement de groupements, tels que définis aux paragraphes 1 et/ou 2 et/ou 3 liés entre eux par un lien valentiel et/ou par un des groupes suivants :

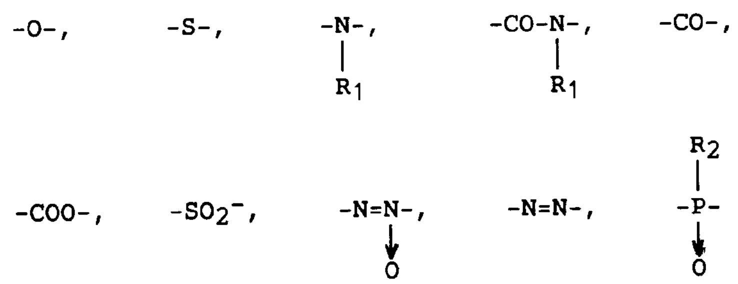

dans ces formules, $R_1$ et $R_2$ représentent un atome d'hydrogène, un radical alkyle ayant de 1 à 4 atomes de carbone, un radical cyclohexyle ou phényle et $R_1$ pouvant représenter un atome d'hydrogène dans la mesure où l'atome d'azote ne se quaternise pas.

Dans la formule (I), le radical R peut porter n'importe quel substituant dans la mesure où il n'entre pas en compétition avec la réaction souhaitée.

Le nombre de substituants peut être variable, généralement de 1 à 3.

Comme exemples de substituants désignés par Y, on peut mentionner :

– un groupe -OH
– un groupe -COOH
– un groupe -CHO
– un groupe $-NO_2$
– un groupe -C≡N
– un groupe amine non quaternisable ou N- protégé
– un atome d'halogène de préférence fluor, chlore ou brome
– un groupe $-CF_3$

Parmi les composés de formule (I), on met en oeuvre plus particulièrement ceux répondant à la formule (I) dans laquelle le radical R représente :

a) un radical alkyle, alcényle, alcadiényle, alcynyle, linéaire ou ramifié ayant de préférence de 1 à 20 atomes de carbone éventuellement porteur d'un ou plusieurs substituants Y.

Parmi ces radicaux, on peut citer notamment les groupes suivants : méthyle, éthyle, butyle, mono- di- ou trichlorométhyle, α-chloroéthyle, α-bromoéthyle, α-bromopropyle, trifluorométhyle,trifluoro-4,4,4 butyle, hydroxyméthyle, hydroxy-1 éthyle, dihydroxy-1,2 éthyle, vinyle, allyle, dichloro-2,2 vinyle, trichloro-1,2,2 vinyle, propène-2 yle, butène-2 yle, butène-3 yle, méthyl-2 propène-1 yle.

b) un radical cycloalkyle ou cycloalcényle ayant de préférence de 3 à 7 atomes de carbone éventuellement porteur d'un ou plusieurs substituants Y ; lesdits radicaux pouvant être polycycliques du type "pontés", c'est-à-dire que deux cycles ont en commun au moins deux atomes de carbone.

On peut nommer, par exemple, le radical cyclohexyle, le radical cyclohexène-1 yle-1

c) un radical phényle, tolyle, xylyle porteur d'un ou plusieurs substituants Y.

d) un radical hétérocyclique monocyclique saturé, insaturé ou aromatique et comportant en tant qu'hétéroatome un ou plusieurs atomes d'oxygène, d'azote ou de soufre contenant 4 à 6 atomes dans le cycle : les radicaux hétérocycliques pouvant être substitués par un ou plusieurs substituants Y.

A titre illustratif de radicaux R, on peut citer les radicaux pyrrolidinyle, imidazolidinyle, pipéridyle, furyle, pyrrolyle, thiényle, isoxazolyle, furazannyle, isothiazolyle, imidazolyle, pyrazolyle, pyridyle, pyridazinyle, pyrimidinyle.

e) un radical constitué par un enchaînement de 2 à 4 groupes tels que définis aux paragraphes a et/ou b et/ou c et/ou d liés entre eux par un lien valentiel et/ou par au moins un des groupes suivants :

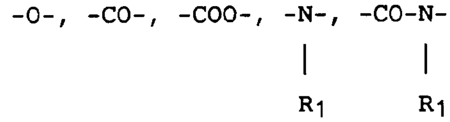

et/ou encore par au moins un groupe $R_3$ de type alcoylène ou alcoylidène ayant de 1 à 4 atomes de carbone, $R_1$ ayant la signification donnée précédemment.

Comme exemples de radicaux résultant d'enchaînement de radicaux, on peut citer tout particulièrement les radicaux du type

$$R_4-A-R_4'-$$

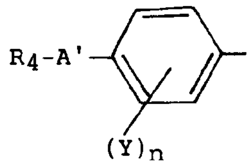

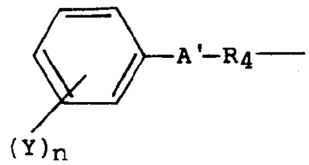

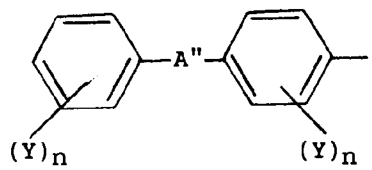

dans lesdites formules:

- $R_4$ et $R'_4$ identiques ou différents représentent un radical aliphatique saturé, linéaire ou ramifié ayant de 1 à 12 atomes de carbone, de préférence de 1 à 6,
- Y représente un substituant ayant la signification donnée précédemment,
- n est un nombre entre 0 et 3,
- A, A′ et A″ identiques ou différents, représentent l'un des groupes suivants :

$$-O-,\ -CO-,\ -COO-,\ -\underset{\displaystyle R_1}{\underset{|}{N}}-,\ -CO-\underset{\displaystyle R_1}{\underset{|}{N}}-$$

A′ et A″ pouvant également représenter un lien valentiel et A″ un groupe $R_3$ de type alcoylène ou alcoylidène ayant de 1 à 4 atomes de carbone, $R_1$ ayant la signification donnée précédemment.

A titre illustratif de ce type de radicaux R, on peut mentionner les radicaux aryle tels que les radicaux biphényle, méthylène-1,1′ biphényle ; les radicaux arylalkyle comme le radical phényl-2 éthyle, le radical [(butyl-2)-4 phényl]-2 éthyle, le radical styryle ; les radicaux cyclo-alkyle tels que le radical cyclohexylméthyle, le radical cyclohexylbutyle, le radical a-phénylcyclohexylméthyle ; les radicaux méthoxyméthyle, phénoxyméthyle, phénoxy-2 éthyle, oxy-1,1′ biphényle, (carboxy-4 phénoxy-4) phényle, méthylènedioxy-3,4 phényle-1, acétyl-2 phényle, acétyl-4 phényle, benzoyl-2 phényle, benzoyl-4 phényle, benzoyloxy-3 éthyl-2 phényle, acétoxy-2 phényle, acétoxy-4 phényle, acétamidométhyle, acétamido-2 phényle, acétamido-4 phényle.

f) un radical aromatique constitué par un ensemble de 2 à 3 cycles aromatiques carbocycliques et/ou hétérocycliques et formant entre eux des systèmes orthocondensés.

Les cycles aromatiques sont de préférence des noyaux benzéniques et pyridiniques.

On donne, ci-après, à titre illustratif des exemples des radicaux aromatiques polycycliques : les radicaux anthryle, quinolyle, naphtyridinyle, benzofurannyle, indolyle.

g) un radical aliphatique, cyclo- ou arylaliphatique tel que défini aux paragraphes a à f, porteur d'au moins une fonction amine N- protégée par un groupe protecteur P et plus particulièrement les groupes suivants : acyle (acétyle, benzoyle), BOC (butyloxycarbonyl), CBZ (carbobenzoxy), FMOC (fluorényl-9 méthoxycarbonyl), MSOC (méthanesulfényl-2 éthoxy carbonyl).

A titre d'acides carboxyliques comprenant au moins un groupe carboxylique répondant à la formule (I), on fait appel plus particulièrement aux composés suivants :

– les acides monocarboxyliques aliphatiques saturés tels que l'acide formique, acétique, propionique, butyrique, isobutyrique, valérique, isovalérique, pivalique, laurique, myristique, palmitique, stéarique,
– les acides dicarboxyliques aliphatiques saturés tels que l'acide oxalique, malonique, succinique, glutarique, adipique, pimélique, subérique, azélaïque, sébacique,
– les acides monocarboxyliques ou dicarboxyliques aliphatiques insaturés tels que l'acide acrylique, propiolique, méthacrylique, crotonique, isocrotonique, oléïque, maléique, fumarique, citraconique, mésaconique,
– les acides carboxyliques carbocycliques saturés ou insaturés tels que l'acide camphorique, l'acide chrysanthémique,
– les acides carboxyliques hétérocycliques tels que les acides furannecarboxyliques, thiophènecarboxyliques, pyrrolecarboxyliques, pyrazinecarboxyliques, l'acide nicotinique, isonicotinique, l'acide picolinique,
– les acides carboxyliques carbocycliques aromatiques tels que l'acide benzoïque, phtalique, isophtalique, téréphtalique, les acides naphtalènecarboxyliques, les acides toluiques,
– les acides carboxyliques arylaliphatiques saturés tels que, notamment les arylpropioniques comme l'acide phényl-2 propionique, l'acide [(butyl-2)-4 phényl]-2 propionique, l'acide (benzoyl-3 phényl)-2 propionique, l'acide (méthoxy-6 naphtyl-2)-2 propionique ou les acides insaturés comme par exemple l'acide phényl-2 propénoïque, l'acide cinnamique,
– les acides carboxyliques halogénés tels que l'acide monochloroacétique, dichloroacétique, trichloroacétique, monochloropropionique, a-bromopropionique, a-bromobutyrique, trifluoroacétique,
– les hydroxy-acides aliphatiques, cycloaliphatiques arylaliphatiques, tels que l'acide glycolique, l'acide lactique, l'acide glycérique, l'acide hydroxy-2 butanoïque, l'acide hydroxy-3 butanoïque, l'acide méthyl-2 lactique, l'acide hydroxy-2 méthylthio-4 butanoïque, l'acide tartronique, l'acide malique, l'acide tartrique, l'acide hydroxy-1 cyclopropane carboxylique, l'acide hydroxy-2 phénylpropanoïque, l'acide hydroxy-2 cinnamique, l'acide hydroxy-3 cinnamique, l'acide hydroxy-4 cinnamique,
– les alcoxy- et phénoxyacides tels que l'acide méthoxyacétique, phénoxyacétique, dichloro-2,4 phénoxyacétique, phénoxypropionique, dichloro-2,4 phénoxypropionique, p-hydroxyphénoxypropionique, m-chlorophénoxypropionique, l'acide phénoxy-4 benzoïque, l'acide (carboxy-4 phénoxy-4) benzoïque, l'acide pipéronylique,
– les oxo-acides tels que l'acide acétyl-2 benzoïque, l'acide acétyl-4 benzoïque, l'acide benzoyl-2 benzoïque, l'acide benzoyl-4 benzoïque,
– les acyloxy-acides tels que l'acide benzoyloxy-3 propionique, l'acide acétoxy-2 benzoïque, l'acide acétoxy-4 benzoïque,
– les amido-acides tels que l'acide acétamido-2 acrylique, l'acide acétamido-2 benzoïque, l'acide acétamido-3 benzoïque, l'acide N-acétamido-4 benzoïque,
– les acides aminés N- protégés par un groupe protecteur comme par exemple les groupes suivants acyle (acétyle, benzoyle), BOC (butyloxycarbonyl), CBZ (carbobenzoxy), FMOC (fluorényl-9 méthoxycarbonyl), MSOC (méthanesulfényl-2 éthoxycarbonyl). On peut citer les acides aminés N-protégés dérivant des acides aminés suivants :
. acides aminés aliphatiques : glycine, alanine, valine, leucine, isoleucine,
. acides aminés hydroxylés : sérine, thréonine,
. acides aminés soufrés : cystéine, méthionine,
. acides aminés dicarboxyliques et leurs amides : acide aspartique, asparagine, acide glutamique, glutamine,
. acides aminés possédant deux groupements basiques : lysine, arginine, histidine,
. acides aminés aromatiques : phénylalanine, tyrosine, tryptophanne,
. imino-acides : proline, hydroxyproline.

Parmi tous les composés cités précédemment à titre illustratif et sans caractère limitatif, le procédé de l'invention s'applique particulièrement bien aux composés suivants :
– l'acide acétique, l'acide propionique et leurs dérivés substitués par un groupe hydroxy, halogène, aryl, aryloxy,
– l'acide benzoïque et ses dérivés substitués par un groupe alkyle $C_1$-$C_4$, acétoxy, acétamido,
– l'acide nicotinique,
– les acides aminés N-protégés.

Le procédé de l'invention consiste donc à mettre en contact au moins un des acides carboxyliques précités avec au moins un dérivé halogéné que l'on peut représenter symboliquement par la formule (II)

$$R_5\text{-}X \quad \text{(II)}$$

dans ladite formule (II):
– X est un atome d'halogène : ledit atome d'halogène ne devant pas être en position vinylique ou alcynyle

– $R_5$, éventuellement substitué, représente un radical aliphatique acyclique, saturé ou insaturé, linéaire ou ramifié ; un radical cycloaliphatique, saturé ou insaturé, monocylique ou polycyclique ; un radical cyclo- ou arylaliphatique saturé ou insaturé, linéaire ou ramifié.

L'atome d'halogène peut être le chlore, le brome ou l'iode mais on choisit, de préférence, les deux premiers.

Le radical $R_5$ représente plus particulièrement :

1°- un radical alkyle, alcényle, alcadiényle, alcynyle linéaire ou ramifié ayant, de préférence de 1 à 12 atomes de carbone ; la chaîne hydrocarbonée pouvant être éventuellement interrompue par un des groupes suivants :

$$-O-,\ -CO-,\ -COO-,\ -\underset{\underset{R_1}{|}}{N}-,\ -CO-\underset{\underset{R_1}{|}}{N}-$$

dans lesdites formules, $R_1$ ayant la signification donnée précédemment ou être porteuse d'un ou plusieurs substituants Y tels que précités.

2° - un radical cycloalkyle ou cycloalcényle ayant de préférence de 5 à 7 atomes de carbone éventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

$$R_1-CO-,\ R_1-COO-,\ R_1-O-CO-,\ R_1-\underset{\underset{R_1}{|}}{N}-,\ R_1-CO-\underset{\underset{R_1}{|}}{N}-,\ R_1-\underset{\underset{R_1}{|}}{N}-CO-$$

dans lesdites formules, $R_1$ ayant la signification donnée précédemment ; lesdits radicaux pouvant être polycycliques de type "pontés".

3° - un radical aliphatique acyclique, saturé ou insaturé, linéaire ou ramifié, tel que précisé sous 1°, porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes suivants :

$$-O-,\ -CO-,\ -COO-,\ -\underset{\underset{R_1}{|}}{N}-,\ -CO-\underset{\underset{R_1}{|}}{N}-$$

dans lesdites formules, $R_1$ ayant la signification donnée précédemment.

Comme exemples de cycles, on peut envisager:

a) un radical cycloaliphatique saturé ou insaturé, monocyclique ou polycyclique tel que mentionné sous 2°.

b) un radical phényle, tolyle, xylyle, éventuellement porteur d'un ou plusieurs substituants Y ou Z.

c) un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle : ledit radical pouvant être porteur d'un ou plusieurs substituants Y ou Z.

d) un radical constitué par un enchaînement de 2 à 4 groupes tels que définis aux paragraphes a et/ou b et/ou c, liés entre eux par un lien valentiel et/ou par un des groupes suivants :

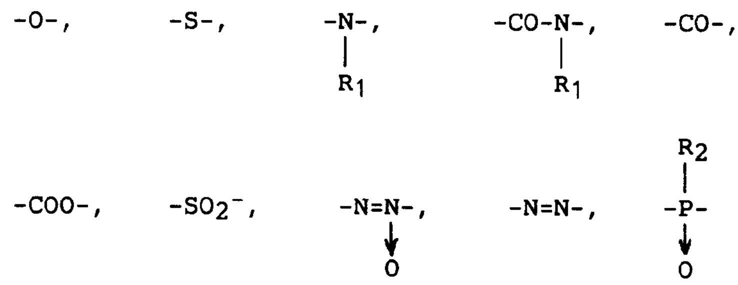

et/ou encore au moins un groupe $R_3$ de type alcoylène ou alcoylidène ayant de 1 à 4 atomes de carbone ; dans lesdites formules $R_1$ et $R_2$ ayant la signification donnée précédemment.

e) un radical aromatique constitué par un ensemble de 2 à 3 cycles aromatiques carbocycliques et/ou hétérocycliques et formant entre eux des systèmes orthocondensés.

Dans la définition du radical $R_5$ du dérivé halogéné de formule (II), il est noté que celui-ci peut porter de un à plusieurs substituants Y ou Z. Par "plusieurs, on entend généralement un nombre total ne dépassant pas 3.

D'une manière préférentielle, le radical $R_5$ représente:

– un radical alkyle linéaire ou ramifié ayant de 1 à 8 atomes de carbone, tel que par exemple, le radical méthyle, éthyle, propyle, isopropyle, butyle, isobutyle, sec-butyle, tert-butyle, éthyl-2 butyle, pentyle, isopentyle, néopentyle, tert-pentyle, méthyl-2 pentyle, hexyle, éthyl-2 hexyle,

– un radical alcényle linéaire ou ramifié ayant de 2 à 8 atomes de carbone et comportant une double liaison éthylénique en β du groupe X comme par exemple, le radical allyle, méthyl-3 butène-2 yle, hexène-3 yle,

– un radical cyclohexyle éventuellement substitué par un radical alcoyle linéaire ou ramifié ayant de 1 à 4 atomes de carbone,

– un radical arylalkyle tel que symbolisé par la formule suivante

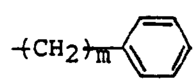

dans laquelle m est un nombre entier allant de 1 à 4, de préférence, égal à 1.

Parmi les dérivés halogénés répondant à la formule (II), on peut citer tout particulièrement, les composés suivants :

– le bromure de méthyle
– le bromure d'éthyle
– le bromure de n-propyle
– le bromoisopropane
– le bromohexane
– le bromoheptane
– le bromooctane
– le bromo-1 méthyl-2 butane
– le bromo-1 méthyl-3 butane
– le bromo-2 méthyl-2 butane
– le bromure d'allyle
– le chlorure d'allyle
– le chlorure de crotyle
– le chloro-3 méthyl-2 propène
– le chloro-1 butène-2
– le chloro-1 méthyl-3 butène-2
– le bromure de prényle
– le bromure de géranyle
– le chlorure de géranyle
– le bromocyclobutane
– le bromocyclopentane
– le bromocyclohexane

– le bromocycloheptane
– le (bromométhyl)cyclopropane
– le (chlorométhyl-2)pyridine
– le (chlorométhyl-3)pyridine
– le (chlorométhyl-4)pyridine
– le bromure de benzyle
– le chlorure de benzyle
– le chlorométhyl-4 stilbène
– le (bromométhyl-1) naphtalène
– le (bromométhyl-2) naphtalène

En ce qui concerne le choix de l'halogénure utilisé, soit chlorure ou bromure, on le détermine en fonction de la nature de l'hydrocarbure dont dérive l'halogénure et d'impératifs économiques.

On peut faire appel dans la mise en oeuvre du procédé de l'invention, à un composé bromé dans tous les cas.

Toutefois, les chlorures étant les moins chers, on cherchera à les utiliser préférentiellement, mais ceux-ci ne peuvent pas toujours être utilisés. En effet, il n'est possible selon l'invention d'avoir recours à un halogénure de type chlorure que s'il y a présence d'une insaturation sur la chaîne hydrocarbonée portant l'atome de chlore, en β par rapport à ce dernier : l'insaturation peut être présente sur une chaîne aliphatique ou dans un cycle aromatique. Par exemple, le chlorure de benzyle peut être avantageusement utilisé.

Il a été mentionné dans la définition du radical $R_5$ que celui-ci pouvait porter un autre atome d'halogène.

Il devient ainsi possible selon le procédé de l'invention d'obtenir des diesters d'acides carboxyliques.

Il y a lieu de remarquer que les atomes d'halogène ne doivent pas être présents sur le même atome de carbone.

Des diesters d'un acide carboxylique peuvent être obtenus dans le cas de la mise en oeuvre :

– de dérivés dibromés quels qu'ils soient, tels que par exemple, le dibromométhane, le dibromo-1,2 éthane, le dibromo-1,2 propane, le dibromo-1,3 propane, le dibromo-1,2 butane, le dibromo-1,3 butane, le dibromo-1,4 butane, le dibromo-1,6 hexane, le dibromo-1,4 butène-2, le dibromo-1,4 butyne, le bis-(bromométhyl)-1,4 benzène,

– de dérivés dichlorés présentant une insaturation en position β par rapport aux atomes de chlore comme, par exemple, le dichloro-1,4 pentène-2, le bis-(chlorométhyl)-1,4 benzène,

– de dérivés mixtes bromé et chloré dans la mesure où il y a une insaturation en position β par rapport à l'atome de chlore. Dans le cas contraire, par exemple lors de la mise en oeuvre du bromochloroéthane, il ne sera pas possible d'obtenir un diester, mais seulement un mono-ester β-chloré.

Selon le procédé de l'invention, il est également possible d'obtenir un diester lorsque le radical R de l'acide carboxylique de formule (I) présente à titre de substituant Y, une autre fonction -COOH.

Intervient également dans le procédé de l'invention, une amine tertiaire non quaternisable par le dérivé halogéné. Elle sert à neutraliser l'acide halohydrique libéré au cours de l'estérification.

Les amines utilisées comme catalyseur dans le présent procédé sont des amines tertiaires dont au moins l'un des radicaux portés par l'azote, est un radical aliphatique ramifié, et de préférence dont au moins deux des radicaux sont un radical aliphatique ramifié.

Comme exemples d'amines convenant à l'invention, on peut citer celles qui répondent la formule (III) suivante :

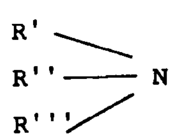 (III)

– dans laquelle R′, R″ et R‴ identiques ou différents représentent:
  . un radical aliphatique linéaire ou ramifié, saturé ou insaturé ayant de 1 à 12 atomes de carbone,
  . un radical cycloaliphatique saturé ou insaturé ayant de 5 à 7 atomes de carbone,
  . un radical phényle.
– dans ladite formule (III) :
  . au moins l'un des radicaux R′, R″ et R‴ représentant un radical aliphatique ramifié.
  . au plus l'un des radicaux R′, R″ et R‴ est un radical phényle.

A titre de radical ramifié, on choisit, de préférence, un radical aliphatique ramifié présentant une ramification sur le carbone en position a par rapport à l'atome d'azote.

Parmi les composés de formule (III), on choisit préférentiellement ceux répondant à la formule (III) ayant au moins deux des radicaux R′, R″ et R‴ aliphatiques ramifiés avec des ramifications situées de préférence sur le carbone en position a par rapport à l'atome d'azote.

A titre d'exemples de radicaux R′, R″ et R‴, on peut mentionner les radicaux méthyle, éthyle, propyle, isopropyle, butyle, isobutyle, sec-butyle, tert-butyle, pentyle, isopentyle, néopentyle, tert-pentyle, hexyle, isohexyle, octyle, isooctyle, décyle, dodécyle, cyclohexyle, phényle.

Comme exemples d'amines tertiaires convenant bien à l'invention, on peut citer :

- la diisopropylméthylamine
- la N,N-diisopropyléthylamine
- la N,N-diisopropyl n-propylamine
- la N,N-di sec-butyléthylamine
- la N,N-diisobutyléthylamine
- la N,N,N-triisopropylamine
- la N,N-diisopropylallylamine
- la N,N-di sec-butylallylamine
- la N,N-dicyclohexyléthylamine
- le N,N-diisopropylamino-2 éthanol

On préfère utiliser dans le procédé de l'invention une base présentant un pka mesuré à 25°C supérieur ou égal à 9,0 et, de préférence, supérieur ou égal à 10.

On choisit préférentiellement parmi l'ensemble des bases citées, la N,N-diisopropyléthylamine.

De plus, on préfère faire appel à une amine tertiaire non quaternisable insoluble dans l'eau car cela facilite sa récupération, en fin de réaction et évite la consommation parasite du dérivé halogéné.

Une variante préférentielle du procédé de l'invention consiste à introduire également dans le milieu réactionnel un catalyseur du type onium car il permet d'accroître la cinétique réactionnelle.

Les oniums utilisés dans le procédé de l'invention sont ceux dérivant notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du sélénium, de l'oxygène, du carbone ou de S=O seront tricoordinés tandis que les ions onium dérivant de l'iode seront dicoordinés.

Les restes hydrocarbonés coordinés à ces différents éléments sont des radicaux alkyles, alcényles, aryles, cycloalkyles, aralkyles éventuellement substitués, 2 restes hydrocarbonés coordinés pouvant former ensemble un radical unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581 - 587 (1968), les termes "dure" et "intermédiaire" ayant respectivement la signification des termes de "hard" et "borderline" utilisés dans cette référence.

Parmi les ions onium pouvant être utilisés dans le présent procédé de l'invention, conviennent particulièrement ceux répondant à l'une des formules générales suivantes :

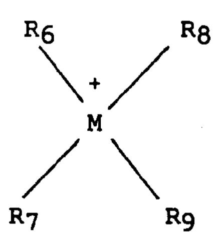

(IV)

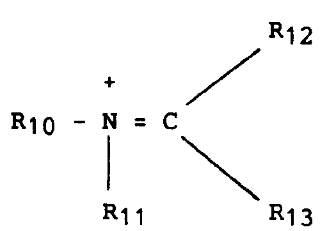

(V)

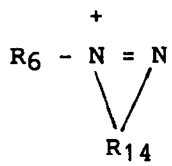

(V bis)

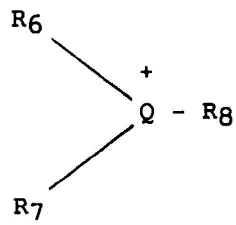

(VI)

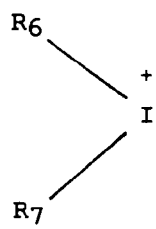

(VII)

dans lesquelles:

- M représente N, P ou As ;
- Q représente S, O, Se, S=O ou C ;
- $R_6$, $R_7$, $R_8$ et $R_9$, identiques ou différents représentent :
  - . un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
  - . un radical alcényle, linéaire ou ramifié, ayant 2 à 12 atomes de carbone ;
  - . un radical aryle ayant 6 à 10 atomes de carbone, éventuellement substitué par un ou plusieurs groupements ou atomes alkyle ayant 1 à 4 atomes de carbone, alkoxy, alkoxycarbonyle, le radical alkoxy ayant 1 à 4 atomes de carbone, ou halogène,
  - . deux desdits radicaux $R_8$ à $R_9$ pouvant former ensemble un radical alkylène, alcénylène ou alcadiènylène, linéaire ou ramifié ayant de 3 à 6 atomes de carbone ;
- $R_{10}$, $R_{11}$, $R_{12}$, $R_{13}$ sont identiques ou différents et représentent :
  - . un radical alkyle linéaire ou ramifié contenant de 1 à 4 atomes de carbone ;
  - . les radicaux $R_{12}$ et $R_{13}$ pouvant former ensemble un radical alkylène contenant de 3 à 6 atomes de carbone ;
  - . les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;
- $R_{14}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_6$.

Parmi les bases "dures" ou "intermédiaires" pouvant constituer l'anion desdits sels d'onium, on peut citer les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, Ph-$SO_3^-$, $HSO_4^-$, $NO_3^-$, $SO_4^{2-}$, $Cl^-$, $Br^-$, $I^-$, $OH^-$, Ph représentant un radical phényle, ainsi que tous les autres anions répondant à la définition de base "dure" ou "intermédiaire" de PEARSON.

Pour des raisons de commodité de mise en oeuvre, lesdits anions pourront être choisis parmi $PO_4^{3-}$, $HPO_4^{2-}$, $H_2PO_4^-$, $CH_3SO_3^-$, Ph-$SO_3^-$, $NO_3$-, $SO4^{2-}$, $PF_6^-$, $Cl^-$, $Br^-$, $I^-$, Ph ayant la signification précédente. On recourra avantageusement aux anions $Br^-$ et $I^-$.

A titre d'exemples de cations organiques répondant à la formule (IV) on peut citer les cations :

- tétraméthylammonium,
- triéthylméthylammonium,
- tributylméthylammonium,
- triméthylpropylammonium,
- tétraéthylammonium,
- tétrabutylammonium,
- dodécyltriméthylammonium,
- méthyltrioctylammonium,
- heptyltributylammonium,
- tétrapropylammonium,
- tétrapentylammonium,
- tétrahexylammonium,
- tétraheptylammonium,
- tétraoctylammonium,
- tétradécylammonium,
- butyltripropylammonium,
- méthyltributylammonium,
- pentyltributylammonium,
- méthyldiétylpropylammonium,
- éthyldiméthylpropylammonium,
- tétradodécylammonium,
- tétraoctadécylammonium,
- hexadécyltriméthylammonium,
- benzyltriméthylammonium,
- benzyldiméthylpropylammonium,
- benzyldiméthyloctylammonium,
- benzyltributylammonium,
- benzyltriéthylammonium,
- phényltriméthylammonium,
- benzyldiméthyltétradécylammonium,
- benzyldiméthylhexadécylammonium,
- diméthyldiphénylammonium,
- méthyltriphénylammonium,
- butène-2-yltriéthylammonium,
- N,N-diméthyl-tétraméthylèneammonium,
- N,N-diéthyl-tétraméthylèneammonium,
- tétraméthylphosphonium,
- tétrabutylphosphonium,
- éthyltriméthylphosphonium,
- triméthylpentylphosphonium,
- octyltriméthylphosphonium,
- dodécyltriméthylphosphonium,
- triméthylphénylphosphonium,
- diéthyldiméthylphosphonium,
- dicyclohexyldiméthylphosphonium,
- diméthyldiphénylphosphonium,
- cyclohexyltriméthylphosphonium,
- triéthylméthylphosphonium,
- méthyltri(isopropyl)phosphonium,
- méthyltri(n-propyl)phosphonium,
- méthyltri(n-butyl)phosphonium,
- méthyltri(méthyl-2 propyl)phosphonium,
- méthyltricyclohexylphosphonium,
- méthyltriphénylphosphonium,
- méthyltribenzylphosphonium,
- méthyltri(méthyl-4 phényl) phosphonium,
- méthyltrixylylphosphonium,
- diéthylméthylphénylphosphonium,

- dibenzylméthylphénylphosphonium,
- éthyltriphénylphosphonium,
- tétraéthylphosphonium,
- éthyltri(n-propyl)phosphonium,
- triéthylpentylphosphonium,
- hexadécyltributylphosphonium,
- éthyltriphénylphosphonium,
- n-butyltri(n-propyl)phosphonium,
- butyltriphénylphosphonium,
- benzyltriphénylphosphonium,
- (β-phényléthyl)diméthylphénylphosphonium,
- tétraphénylphosphonium,
- triphényl(méthyl-4 phényl)phosphonium,
- tétrakis(hydroxyméthyl)phosphonium,
- tétraphénylarsonium.

Parmi les cations répondant aux formules (V) et (V bis) on peut citer les cations :

- N-méthylpyridinium,
- N-éthylpyridinium,
- N-hexadécylpyridinium,
- N-méthylpicolinium,
- triphényl-1,2,4 triazolium

A titre d'exemples de cations organiques répondant à la formule (VI), on peut citer les cations :

- triméthylsulfonium,
- triéthylsulfonium,
- triphénylsulfonium,
- triméthylsulfoxonium,
- triphénylcarbénium,
- triéthyloxonium.

A titre d'exemples de cations organiques répondant à la formule (VII), on peut citer les cations :

- diphényliodonium,
- diméthoxy-4,4′ diphényliodonium (ou les composés décrits dans JACS 1958, 81, 342),
- diphényliodonium 2-carboxylate

Parmi les oniums qui peuvent être utilisés dans le cadre du présent procédé, on préférera le plus souvent les ions ammonium quaternaire, les ions phosphonium quaternaire, les ions sulfonium et les ions iodonium. Conviennent tout particulièrement bien, les ions ammonium dont les quatre groupes sont des radicaux alkyle identiques possédant de quatre à cinq atomes de carbone.

En ce qui concerne le choix du cation, on préférera $Br^-$ ou $I^-$.

L'onium peut être soluble dans le milieu réactionnel, on aura alors une réaction en milieu homogène.

Il peut être sous forme solide, insoluble et on aura alors une réaction en milieu biphasique solide-liquide. Il en est ainsi notamment dans le cas où l'onium peut être supporté sur une résine minérale ou polymérique.

Comme exemples d'oniums supportés, on peut citer:

- le fluorure de tétrabutylammmonium sur gel de silice,
- le chlorure de tributylammonium sur polymère commercialisé, par exemple par la Société FLUKA,
- le chlorure de méthyltributylphosphonium lié à du polystyrène commercialisé par exemple par la Société FLUKA,
- le bromure de benzyltriméthylammonium sur polymère.

L'onium intervenant dans le procédé de l'invention peut être apporté totalement synthétisé dans le milieu réactionnel ou être préparé "in situ". Dans ce dernier cas, on peut préparer l'onium en mettant en oeuvre une quantité catalytique d'une amine tertiaire quaternisable qui en présence du dérivé halogéné de formule (II), conduit à l'ammonium désiré. Cette amine est, de préférence, la triéthylamine. Un autre mode d'obtention d'un onium consiste à générer in situ l'halohydrate de l'amine tertiaire non quaternisable par le dérivé halogéné de formule (II), en faisant réagir celle-ci avec une quantité catalytique d'un acide halohydrique qui est de préférence, l'acide bromhydrique ou l'acide chlorhydrique. On peut mettre en oeuvre ledit acide halohydrique sous forme de préférence d'une solution concentrée ou bien faire barboter celui-ci sous forme gazeuse dans le milieu réactionnel.

Conformément au procédé de l'invention, la réaction d'estérification de l'acide carboxylique est effectuée en présence d'une amine tertiaire non quaternisable et éventuellement d'un onium, les différents réactifs étant mis en oeuvre généralement dans les proportions définies ci-après.

Le rapport molaire entre le nombre de fonctions carboxyliques de l'acide carboxylique et le nombre d'atomes d'halogène réagissants du dérivé halogéné varie de préférence entre 0,9 et 2,0. Il est choisi préférentiellement aux environs de 1,0.

Quand l'onium est formé in situ, à partir d'une amine tertiaire quaternisable et du dérivé halogéné, on veillera à mettre en oeuvre celui-ci en une quantité excédant la quantité stoechiométrique.

En ce qui concerne la quantité d'amine tertiaire non quaternisable, celle-ci est déterminée de telle sorte qu'elle piège l'acide halohydrique formé au cours de la réaction. Elle peut donc varier de la quantité stoechiométrique jusqu'à un excès représentant environ 500 % dans le cas où ladite amine est utilisée comme solvant.

Si l'onium est formé in situ à partir de l'amine tertiaire non quaternisable et d'un acide halohydrique, il y a lieu de prévoir un excès par rapport à la stoechiométrie.

Quant à l'onium, il est mis en oeuvre en une quantité catalytique, c'est-à-dire en une quantité telle que le rapport molaire entre l'onium et l'acide carboxylique soit situé, de préférence, entre 0,025 et 0,2.

Comme mentionné précédemment, la réaction peut être conduite en l'absence de tout solvant ou bien en présence d'un solvant organique.

Le choix du solvant est déterminé en fonction de son aptitude à solubiliser les réactifs, à savoir l'acide carboxylique et le dérivé halogéné. De plus, il doit être inerte dans les conditions réactionnelles.

Il n'est pas nécessaire mais souhaitable que le solvant choisi solubilise l'amine tertiaire non quaternisable et l'onium.

Un mode de réalisation préférentiel de l'invention consiste à utiliser l'amine tertiaire non quaternisable comme solvant réactionnel. On choisit, de préférence, la N,N-diisopropyléthylamine.

Comme exemples de solvants réactionnels, on peut faire appel en particulier aux hydrocarbures aliphatiques ou aromatiques, aux hydrocarbures halogénés aliphatiques ou aromatiques, aux esters aliphatiques, à certains solvants aprotiques polaires, tels que les carboxamides linéaires ou cycliques comme le diméthylformamide, le diéthylformamide ou la N-méthylpyrrolidone ; les nitriles aliphatiques ou aromatiques comme l'acétonitrile, le propionitrile ou le benzonitrile.

A titre d'hydrocarbures aliphatiques ou aromatiques, on peut citer l'hexane, l'heptane, l'octane, le nonane, le décane ou le cyclohexane, le toluène, le xylène.

A titre d'hydrocarbures halogénés aliphatiques ou aromatiques, on peut mentionner : les hydrocarbures perchlorés tels que notamment le tétrachlorure de carbone, le tétrachloroéthylène, l'hexachloroéthane, l'hexachloropropène et l'hexachlorobutadiène, les hydrocarbures partiellement chlorés tels que le chlorure de méthylène, le dichloroéthane, le tétrachloroéthane, le trichloroéthylène, le chloro-1 butane, le dichloro-1,2 butane ; le monochlorobenzène, le dichloro-1,2 benzène, le dichloro-1,3 benzène, le dichloro-1,4 benzène ou des mélanges de différents chlorobenzènes ; le monobromobenzène ou des mélanges de monobromobenzène avec un ou plusieurs dibromobenzènes.

Lorsque le procédé de l'invention est mis en oeuvre en milieu solvant, la concentration de l'acide carboxylique n'est pas critique. Elle dépendra notamment de la solubilité de celui-ci dans le solvant organique utilisé.

Selon un mode de réalisation pratique de l'invention, on peut effectuer le mélange de tous les réactifs dans un ordre quelconque.

Une réalisation préférentielle de l'invention consiste à préparer un pied de cuve comprenant l'acide carboxylique, l'amine tertiaire non quaternisable, éventuellement l'onium et un solvant organique.

On effectue ensuite l'addition généralement progressive du dérivé halogéné dans le milieu réactionnel.

La température à laquelle est mise en oeuvre le procédé de l'invention est généralement inférieure ou égale à 120°C. Cette limite est imposée par des contraintes économiques car le rendement de la réaction décroît au-delà de cette température. La température limite inférieure se situe aux environs de 50°C. Celle-ci ne présente aucun caractère critique.

D'une manière préférentielle, la température est choisie entre 70°C et 120°C.

La pression réactionnelle n'est pas critique et est généralement la pression atmosphérique.

Pour atteindre les températures indiquées précédemment et ne pas perdre de solvant, on travaille de préférence sous pression autogène.

La durée de la réaction peut varier dans de larges limites par exemple de 15 minutes à 8 heures, mais elle est le plus souvent de 1 ou 2 heures.

En fin de réaction, l'ester de l'acide carboxylique est séparé par les techniques classiques de séparation, telles que l'extraction par un solvant, le solubilisant par exemple, l'acétate d'éthyle, la méthylisobutylcétone, et/ou la distillation.

Si l'estérification est conduite dans un solvant organique, on dilue à l'eau le milieu réactionnel. On sépare alors la phase organique comprenant l'ester formé et le dérivé halogéné en excès et la phase aqueuse qui contient l'halohydrate de l'amine tertiaire non quaternisable, éventuellement l'onium.

On peut alors récupérer l'amine tertiaire non quaternisable en traitant la phase aqueuse avec une solution

basique, de préférence la soude.

On peut ensuite la recycler.

Une autre variante de récupération de l'ester formé consiste à neutraliser par une base, de préférence, la soude, l'halohydrate de l'amine tertiaire non quaternisable.

Le sel halogéné du cation de la base de préférence NaCl ou NaBr, se trouve en phase aqueuse qui peut être séparée d'une phase organique comprenant l'ester formé, le dérivé halogéné dans le cas d'un excès et l'amine tertiaire non quaternisable.

On peut purifier l'ester par extraction à l'aide d'un solvant organique et/ou par distillation.

Il est à noter que le premier mode de traitement du milieu réactionnel est préféré, car il permet d'accéder plus directement à un ester pur.

Le procédé de l'invention convient tout-à-fait bien à la préparation des esters d'alkyle, de cyclohexyle et de benzyle des acides acétique, propionique et leurs dérivés substitués par un groupe hydroxy, halogène, aryl, aryloxy ; de l'acide benzoïque et de ses dérivés substitués par un groupe alkyle $C_1$-$C_4$, acétoxy, acétamido ; de l'acide nicotinique et des acides aminés N- protégés.

Un des intérêts du procédé de l'invention est qu'il permet de préparer des esters d'acides carboxyliques difficiles à estérifier.

De plus, lorsque l'amine tertiaire est utilisée comme solvant réactionnel, il est possible d'enchaîner d'autres réactions dans le cas de synthèse multi-phases.

Les exemples qui suivent illustrent l'invention sans toutefois la limiter.

Dans les exemples, les abréviations suivantes signifient

$$TT_H = \frac{\text{nombre de moles de dérivé halogéné transformées}}{\text{nombre de moles de dérivé halogéné introduites}}$$

$$TT_A = \frac{\text{nombre de moles d'acide carboxylique transformées}}{\text{nombre de moles d'acide carboxylique introduites}}$$

$$RR_H = \frac{\text{nombre de moles d'ester d'acide carboxylique formées}}{\text{nombre de moles de dérivé halogéné introduites}}$$

$$RR_A = \frac{\text{nombre de moles d'ester d'acide carboxylique formées}}{\text{nombre de moles d'acide carboxylique introduites}}$$

Les exemples suivants illustrent la préparation d'esters d'acides carboxyliques aliphatiques (exemples 1,3,4) et hétérocycliques (exemple 2).

Les exemples 5 à 7 illustrent la mise en oeuvre d'un onium.

EXEMPLE 1

Dans un réacteur en verre de 30 ml muni d'un système d'agitation, d'un dispositif de contrôle de température, d'un réfrigérant et équipé d'un système de chauffage, on charge :

– 0,615 g de bromure d'isopropyle (5 mM)
– 0,71 g de N,N-diisopropyléthylamine (5,5 mM) dénommée de manière simplifiée D.I.P.E.A.

On introduit ensuite progressivement en 1 heure, 0,57 g d'acide trifluoroacétique (5 mM). La réaction étant exothermique, on contrôle la température de telle sorte qu'elle ne dépasse pas 50°C.

Ensuite, on maintient le mélange réactionnel sous agitation et à 50°C, pendant 4 heures.

En fin de réaction et après refroidissement, on ajoute 15 ml d'eau.

On sépare les phases organique et aqueuse.

On extrait la phase organique par 15 ml de chlorure de méthylène.

Le bromure d'isopropyle non réagi et le trifluoroacétate d'isopropyle sont dosés par chromatographie en phase gazeuse (CPG).

Les résultats obtenus sont consignés dans le Tableau I.

Tableau I

| Référence exemple | Amine tertiaire non quaternisable | Température (°C) | $TT_H$ bromure d'isopropyle | $RR_H$ trifluoroacétate d'isopropyle |
|---|---|---|---|---|
| 1 | D.I.P.E.A. | 50 | 81,8 | 75 |

EXEMPLE 2

Dans un réacteur de 30 ml tel que décrit dans l'exemple 1, on charge :
– 0,99 g d'acide nicotinique (8 mM)
– 1,01 g de chlorure de benzyle (8 mM)
– 1,03 g de N,N-diisopropyléthylamine (8 mM)
On agite le mélange réactionnel et on le porte à une température de 70°C.
La durée totale du chauffage est de 5 heures.
En fin de réaction et après refroidissement, on ajoute 10 ml d'acétate d'éthyle puis 8 ml de soude 1N.
On sépare les phases organique et aqueuse.
On dose le chlorure de benzyle et le nicotinate de benzyle par CPG.
On rassemble les résultats obtenus dans le Tableau II.

Tableau II

| Référence exemple | Amine tertiaire non quaternisable | Température (°C) | $TT_H$ chlorure de benzyle | $RR_H$ nicotinate de benzyle |
|---|---|---|---|---|
| 2 | D.I.P.E.A. | 70 | 92,4 | 80 |

EXEMPLE 3

On opère comme dans l'exemple 2, en chargeant :
– 0,51 g d'acide crotonique (5,9 mM)
– 0,97 g de chlorure de benzyle (7,7 mM)
– 1,03 g de N,N-diisopropyléthylamine (8 mM)
La température réactionnelle est de 70°C.
La durée de chauffage est de 5 heures.
On consigne les différents résultats dans le Tableau III.

Tableau III

| Référence exemple | Amine tertiaire non quaternisable | Température (°C) | $TT_A$ acide crotonique | $TT_H$ chlorure de benzyle | $RR_A$ butène-2 oate de benzyle |
|---|---|---|---|---|---|
| 3 | D.I.P.E.A. | 70 | 85 | 67 | 82 |

EXEMPLE 4

On opère comme dans l'exemple 2, en chargeant :
– 3,62 g d'acide L-lactique en solution aqueuse à 20 % (soit 8 mM d'acide pur)
– 1,04 g de chlorure de benzyle (8,2 mM)
– 1,1 g de N,N-diisopropyléthylamine (8,5 mM)
La température réactionnelle est de 100°C.
La durée de chauffage est de 5 heures.
Les résultats obtenus sont consignés dans le Tableau IV.

Tableau IV

| Référence exemple | Amine tertiaire non quaternisable | Température (°C) | $TT_H$ chlorure de benzyle | $RR_H$ lactate de benzyle |
|---|---|---|---|---|
| 4 | D.I.P.E.A. | 100 | 83 | 70 |

Exemples 5 à 7

Influence de la présence d'un onium

On effectue la réaction d'estérification de l'acide sénécioïque (ou acide diméthyl-3,3 acrylique), en l'absence d'onium (exemple 5) et dans une série d'essais, on fait varier la nature de l'onium (exemples 6 et 7).

On opère comme dans l'exemple 2, en chargeant :

- 0,8 g d'acide sénécioïque (8 mM)
- 1,0 g de chlorure de benzyle (8 mM)
- 1,03 g de N,N-diisopropyléthylamine (8 mM)
- x g d'un onium (0,8 mM)

La nature de l'onium utilisé est mentionnée dans le Tableau V.

La température réactionnelle est de 50° C.

La durée totale du chauffage est de 1 heure.

Tableau V

| Référence exemple | Onium | Température (°C) | $TT_H$ chlorure de benzyle | $RR_H$ sénécioate de benzyle |
|---|---|---|---|---|
| 5 | - | 50 | 9,3 | 9,3 |
| 6 | $(Ph)_4\ P^+\ Cl^-$ | 50 | 30,0 | 29,8 |
| 7 | $(Bu)_4\ N^+\ Br^-$ | 50 | 53,0 | 48,0 |

Dans tous les cas, on observe une amélioration du rendement en sénécioate de benzyle.

## Revendications

**1 -** Procédé d'estérification d'un acide carboxylique aliphatique, carbocyclique ou hétérocyclique, cyclo-ou arylaliphatique caractérisé par le fait qu'il consiste à faire réagir ledit acide avec un dérivé halogéné d'un hydrocarbure aliphatique, cycloaliphatique, cyclo- ou arylaliphatique, en phase liquide essentiellement homogène, en présence d'une amine tertiaire non quaternisable.

**2 -** Procédé selon la revendication 1 caractérisé par le fait que l'acide carboxylique répond à la formule (I) suivante :

R-COOH (I)

dans laquelle R, radical hydrocarboné, éventuellement substitué comportant de 1 à 40 atomes de carbone, représente :

1° - un radical aliphatique acyclique, saturé ou insaturé, linéaire ou ramifié,

2° - un radical carbocyclique, saturé, insaturé ou aromatique, monocyclique ou polycyclique.

3° - un radical hétérocyclique, saturé, insaturé ou aromatique, monocyclique ou polycyclique.

4° - un radical monovalent constitué par un enchaînement de groupements, tels que définis aux paragraphes 1 et/ou 2 et/ou 3 liés entre eux par un lien valentiel et/ou par un des groupes suivants :

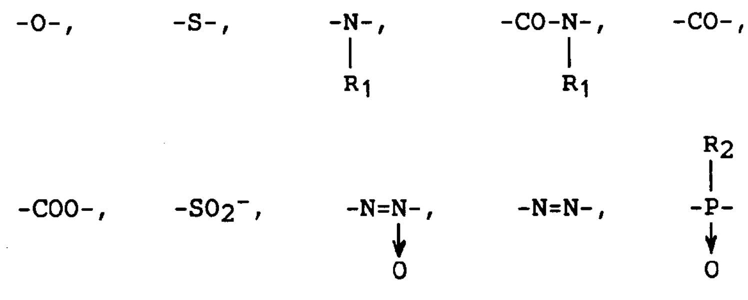

dans ces formules, $R_1$ et $R_2$ représentent un atome d'hydrogène, un radical alkyle ayant de 1 à 4 atomes de carbone, un radical cyclohexyle ou phényle.

**3 -** Procédé selon la revendication 2 caractérisé par le fait que l'acide carboxylique répond à la formule (I) dans laquelle le radical R peut porter de un à trois substituants Y qui peuvent être :

– un groupe -OH
– un groupe -COOH
– un group -CHO
– un group $-NO_2$
– un groupe -C≡N
– un group amine non quaternisable ou N- protégé
– un atome d'halogène de préférence fluor, chlore ou brome
– un group $-CF_3$

**4 -** Procédé selon l'une des revendications 2 et 3 caractérisé par le fait que l'acide carboxylique répond à la formule (I) dans laquelle le radical R représente :

a) un radical alkyle, alcényle, alcadiényle, alcynyle linéaire ou ramifié ayant de préférence de 1 à 20 atomes de carbone éventuellement porteur d'un ou plusieurs substituants Y.

b) un radical cycloalkyle ou cycloalcényle ayant de préférence de 3 à 7 atomes de carbone éventuellement porteur d'un ou plusieurs substituants Y ; lesdits radicaux pouvant être polycycliques du type "pontés".

c) un radical phényle, tolyle, xylyle porteur d'un ou plusieurs substituants Y.

d) un radical hétérocyclique monocyclique saturé, insaturé ou aromatique et comportant en tant qu'hétéroatome un ou plusieurs atomes d'oxygène, d'azote ou de soufre contenant 4 à 6 atomes dans le cycle : les radicaux hétérocyclique pouvant être substitués par un ou plusieurs substituants Y.

e) un radical constitué par un enchaînement de 2 à 4 groupes tels que définis aux paragraphes a et/ou b et/ou c et/ou d liés entre eux par un lien valentiel et/ou par au moins un des groupes suivants :

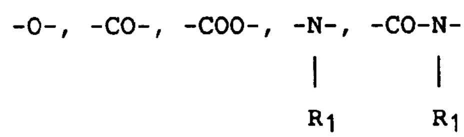

et/ou encore par au moins un groupe $R_3$ de type alcoylène ou alcoylidène ayant de 1 à 4 atomes de carbone.

f) un radical aromatique constitué par un ensemble de 2 à 3 cycles aromatiques carbocycliques et/ou hétérocycliques et formant entre eux des systèmes orthocondensés.

g) un radical aliphatique, cyclo- ou arylaliphatique tel que défini aux paragraphes a à f, porteur d'au moins une fonction amine N- protégée par un group protecteur P et plus particulièrement les groups suivants : acyle (acétyle, benzoyle), BOC (butyloxycarbonyl), CBZ (carbobenzoxy), FMOC (fluorényl-9 méthoxycarbonyl), MSOC (méthanesulfényl-2 éthoxy carbonyl).

**5 -** Procédé selon l'une des revendications 2 à 4 caractérisé par le fait que l'acide carboxylique répond à

la formule (I) dans laquelle le radical R représente l'un des radicaux

$R_4-A-R_4'$—

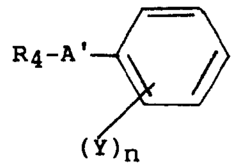

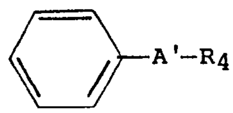

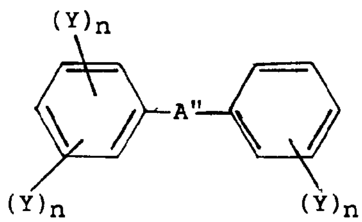

dans lesdites formules:

– $R_4$ et $R'_4$ identiques ou différents représentent un radical aliphatique saturé, linéaire ou ramifié ayant de 1 à 12 atomes de carbone, de préférence de 1 à 6,

– Y représente un substituant ayant la signification donnée précédemment,

– n est un nombre entre 0 et 3,

– A et A′, A″ identiques ou différents, représentent l'un des groupes suivants :

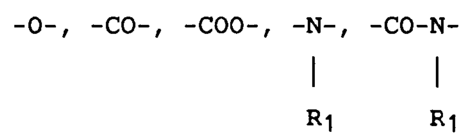

A′ et A″ pouvant également représenter un lien valentiel et A″, un groupe $R_3$ de type alcoylène ou alcoylidène ayant de 1 à 4 atomes de carbone, $R_1$ ayant la signification donnée précédemment.

**6 -** Procédé selon l'une des revendications 1 à 5 caractérisé par le fait que l'acide carboxylique répondant à la formule (I) est choisi parmi les acides carboxyliques suivants :

– les acides monocarboxyliques aliphatiques saturés,
– les acides dicarboxyliques aliphatiques saturés,
– les acides monocarboxyliques ou dicarboxyliques aliphatiques insaturés,
– les acides carboxyliques carbocycliques saturés ou insaturés,
– les acides carboxyliques hétérocycliques
– les acides carboxyliques carbocycliques aromatiques
– les acides carboxyliques arylaliphatiques saturés ou insaturés,
– les acides carboxyliques halogénés,
– les hydroxy-acides aliphatiques, cycloaliphatiques arylaliphatiques,
– les alcoxy- et phénoxyacides,
– les oxo-acides,
– les acyloxy-acides,
– les amido-acides,
– les acides aminés N- protégés par un groupe protecteur comme les groupes suivants acyle, BOC, CBZ, FMOC, MSOC.

**7 -** Procédé selon l'une des revendications 1 à 6 caractérisé par le fait que l'acide carboxylique répondant à la formule (I) est :

– l'acide acétique, l'acide propionique et leurs dérivés substitués par un groupe hydroxy, halogène, aryl, aryloxy,

– l'acide benzoïque et ses dérivés substitués par un groupe alkyle $C_1$-$C_4$, acétoxy, acétamido,

– l'acide nicotinique,

– les acides aminés N-protégés.

**8 -** Procédé selon l'une des revendications 1 à 7 caractérisé par le fait que le dérivé halogéné répond à la formule (II) suivante :

$$R_5\text{-}X \quad (II)$$

dans ladite formule (II) :

– X est un atome d'halogène : ledit atome d'halogène ne devant pas être en position vinylique ou alcynyle

– $R_5$, éventuellement substitué, représente un radical aliphatique acyclique, saturé ou insaturé, linéaire ou ramifié ; un radical cycloaliphatique, saturé ou insaturé, monocylique ou polycyclique ; un radical cyclo- ou arylaliphatique saturé ou insaturé, linéaire ou ramifié.

**9 -** Procédé selon la revendication 8 caractérisé par le fait que le dérivé halogéné répond à la formule (II) dans laquelle le radical $R_5$ représente :

1° - un radical alkyle, alcényle, alcadiényle, alcynyle linéaire ou ramifié ayant, de préférence de 1 à 12 atomes de carbone ; la chaîne hydrocarbonée pouvant être éventuellement interrompue par un des groupes suivants :

$$-O-,\quad -CO-,\quad -COO-,\quad -\underset{\substack{|\\ R_1}}{N}-,\quad -CO-\underset{\substack{|\\ R_1}}{N}-$$

dans lesdites formules, $R_1$ ayant la signification donnée précédemment ou être porteuse d'un ou plusieurs substituants Y tels que précités.

2° - un radical cycloalkyle ou cycloalcényle ayant de préférence de 5 à 7 atomes de carbone éventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

$$R_1-CO-,\quad R_1-COO-,\quad R_1-O-CO-,\quad R_1-\underset{\substack{|\\ R_1}}{N}-,\quad R_1-CO-\underset{\substack{|\\ R_1}}{N}-,\quad R_1-\underset{\substack{|\\ R_1}}{N}-CO-$$

dans lesdites formules, $R_1$ ayant la signification donnée précédemment ; lesdits radicaux pouvant être polycycliques de type "pontés".

3° - un radical aliphatique acyclique, saturé ou insaturé, linéaire ou ramifié, tel que précisé sous 1°, porteur d'un substituant cyclique, ledit radical aliphatique acyclique pouvant être relié au cycle par un lien valentiel ou par l'un des groupes suivants :

$$-O-,\quad -CO-,\quad -COO-,\quad -\underset{\substack{|\\ R_1}}{N}-,\quad -CO-\underset{\substack{|\\ R_1}}{N}-$$

dans lesdites formules, $R_1$ ayant la signification donnée précédemment.

**10 -** Procédé selon l'une des revendications 8 et 9 caractérisé par le fait que le dérivé halogéné répond à la formule (II) dans laquelle $R_5$ représente un radical aliphatique acyclique saturé ou insaturé, linéaire ou ramifié, porteur d'un substituant cyclique qui peut être :

a) un radical cycloaliphatique mono-ou polycyclique tel que défini sous 2° dans la revendication 9.

b) un radical phényle, tolyle, xylyle, éventuellement porteur d'un ou plusieurs substituants Y ou Z.

c) un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle :

ledit radical pouvant être porteur d'un ou plusieurs substituants Y ou Z.

d) un radical constitué par un enchaînement de 2 à 4 groupes tels que définis aux paragraphes a et/ou b et/ou c, liés entre eux par un lien valentiel et/ou par un des groupes suivants :

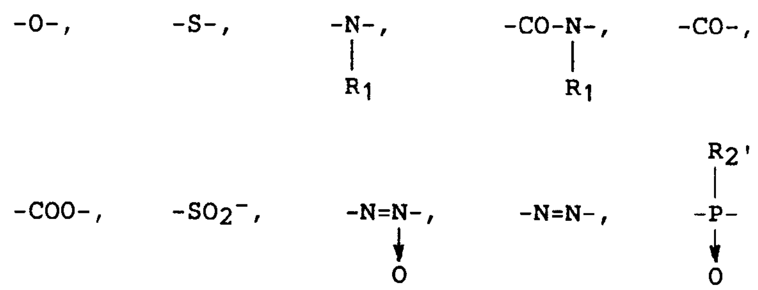

et/ou encore au moins un groupe $R_3$ de type alcoylène ou alcoylidène ayant de 1 à 4 atomes de carbone ; dans lesdites formules $R_1$ et $R_2$ ayant la signification donnée précédemment.

e) un radical aromatique constitué par un ensemble de 2 à 3 cycles aromatiques carbocycliques et/ou hétérocycliques et formant entre eux des systèmes orthocondensés.

**11 -** Procédé selon la revendication 10 caractérisé par le fait que le dérivé halogéné répond à la formule (II) dans laquelle $R_5$ représente:

– un radical alkyle linéaire ou ramifié ayant de 1 à 8 atomes de carbone,

– un radical alcényle linéaire ou ramifié ayant de 2 à 8 atomes de carbone et comportant une double liaison éthylénique en β du groupe X

– un radical cyclohexyle éventuellement substitué par un radical alcoyle linéaire ou ramifié ayant de 1 à 4 atomes de carbone,

– un radical arylalkyle tel que symbolisé par la formule suivante

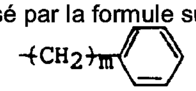

dans laquelle m est un nombre entier allant de 1 à 4.

**12 -** Procédé selon la revendication 11 caractérisé par le fait que le dérivé halogéné est choisi parmi:

– le bromure d'allyle
– le chlorure d'allyle
– le bromure de benzyle
– le chlorure de benzyle
– le bromure d'isopropyle
– le chlorure de crotyle
– le chloro-1 butène-2
– le bromure de cyclohexyle

**13 -** Procédé selon la revendication 1 caractérisé par le fait que l'amine tertiaire non quaternisable est une amine tertiaire dont au moins l'un des radicaux portés par l'azote, est un radical aliphatique ramifié, et de préférence dont au moins deux des radicaux sont un radical aliphatique ramifié.

**14 -** Procédé selon la revendication 13 caractérisé par le fait que l'amine tertiaire non quaternisable répond à la formule (III) suivante :

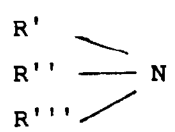
(III)

– dans laquelle R′, R″ et R‴ identiques ou différents représentent :

. un radical aliphatique linéaire ou ramifié, saturé ou insaturé ayant de 1 à 12 atomes de carbone,

. un radical cycloaliphatique saturé ou insaturé ayant de 5 à 7 atomes de carbone,

. un radical phényle.

– dans ladite formule (III) :

. au moins l'un des radicaux R′, R″ et R‴ représentant un radical aliphatique ramifié.

. au plus l'un des radicaux R′, R″ et R‴ est un radical phényle.

**15** - Procédé selon l'une des revendications 13 et 14 caractérisé par le fait que l'amine tertiaire non quaternisable répond à la formule (III) dans laquelle au moins l'un des radicaux R', R″ et R‴ est un radical aliphatique ramifié présentant une ramification sur le carbone en position a par rapport à l'atome d'azote, de préférence au moins deux des radicaux R, R″ et R‴ présentent ladite ramification.

**16** - Procédé selon l'une des revendications 13 à 15 caractérisé par le fait que l'amine tertiaire non quaternisable présente un pka supérieure à 9,0, de préférence supérieur à 10.

**17** - Procédé selon l'une des revendications 13 à 16 caractérisé par le fait que l'amine tertiaire non quaternisable est choisi parmi :

- la diisopropylméthylamine
- la N,N-diisopropyléthylaamine
- la N,N-diisopropyl n-propylamine
- la N,N-di sec-butyléthylamine
- la N,N-diisobutyléthylamine
- la N,N,N-triisopropylamine
- la N,N-diisopropylallylamine
- la N,N-di sec-butylallylamine
- la N,N-dicyclohexyléthylamine
- le N,N-diisopropylamino-2 éthanol

**18** - Procédé selon l'une des revendications 1 à 17 caractérisé par le fait que l'on opère en présence d'un onium répondant à l'une des formules générales suivantes :

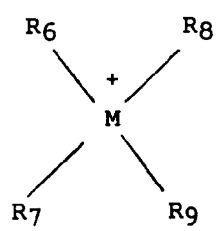

(IV)

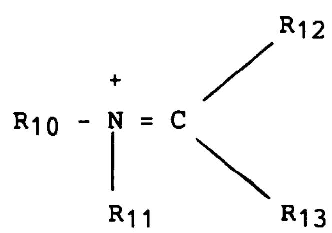

(V)

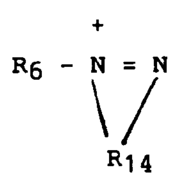

(V bis)

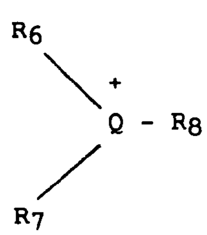

(VI)

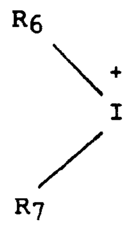

(VII)

dans lesquelles :

– M représente N, P ou As ;

– Q représente S, O, Se, S=O ou C ;

– $R_6$, $R_7$, $R_8$ et $R_9$, identiques ou différents représentent :

. un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;

. un radical alcényle, linéaire ou ramifié, ayant 2 à 12 atomes de carbone ;

. un radical aryle ayant 6 à 10 atomes de carbone, éventuel lement substitué par un ou plusieurs groupements ou atomes alkyle ayant 1 à 4 atomes de carbone, alkoxy, alkoxycarbonyle, le radical alkoxy ayant 1 à 4 atomes de carbone, ou halogène,

. deux desdits radicaux $R_8$ à $R_9$ pouvant former ensemble un radical alkylène, alcénylène ou alcadiènylène, linéaire ou ramifié ayant de 3 à 6 atomes de carbone ;

– $R_{10}$, $R_{11}$, $R_{12}$, $R_{13}$ sont identiques ou différents et représentent :

. un radical alkyle linéaire ou ramifié contenant de 1 à 4 atomes de carbone ;

. les radicaux $R_{12}$ et $R_{13}$ pouvant former ensemble un radical alkylène contenant de 3 à 6 atomes de carbone ;

. les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

– $R_{14}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_6$.

**19** - Procédé selon la revendication 18 caractérisé par le fait que l'anion desdits sels d'onium est choisi parmi les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, Ph-$SO_3^-$, $HSO_4^-$, $NO_3^-$, $SO_4^{2-}$, $Cl^-$, $Br^-$, $I^-$, $OH^-$, Ph représentant un radical phényle ; l'anion desdits sels d'onium étant choisi de préférence parmi les ions $Br^-$ et $I^-$.

**20** - Procédé selon l'une des revendications 18 et 19 caractérisé par le fait que l'onium est un ion ammonium quaternaire, un ion phosphonium quaternaire, un ion sulfonium ou un ion iodonium.

**21** - Procédé selon l'une des revendications 1 à 20 caractérisé par le fait que le rapport molaire entre le nombre de fonctions carboxyliques de l'acide carboxylique et le nombre d'atomes d'halogène réagissants du dérivé halogéné varie entre 0,9 et 2,0 et est, de préférence, égal à 1,0.

**22** - Procédé selon l'une des revendications 1 à 21 caractérisé par le fait que la quantité d'amine tertiaire non quaternisable varie de la quantité stoechiométrique jusqu'à un excès de 500 %.

**23** - Procédé selon l'une des revendications 1 à 22 caractérisé par le fait que le rapport molaire onium/acide carboxylique est compris entre 0,025 et 0,2.

**24** - Procédé selon l'une des revendications 1 à 23 caractérisé par le fait que la réaction est conduite dans un solvant choisi parmi les hydrocarbures aliphatiques ou aromatiques, les hydrocarbures halogénés aliphatiques ou aromatiques, les solvants aprotiques polaires et les amines tertiaires non quaternisables ; le solvant choisi étant de préférence, le diméthylformamide ou la N,N-diisopropyléthylamine.

**25** - Procédé selon l'une des revendications 1 à 24 caractérisé par le fait que la température réactionnelle varie entre 70°C et 120°C.

**26** - Procédé selon l'une des revendications 1 à 25 caractérisé par le fait que l'on ajoute progressivement le dérivé halogéné dans le mélange réactionnel comprenant l'acide carboxylique, l'amine tertiaire non quaternisable, éventuellement un onium et un solvant organique.

Office européen des brevets

# RAPPORT DE RECHERCHE EUROPEENNE

Numero de la demande

EP 91 40 1606

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DE-B-1 152 416 (MONSANTO)<br>* Colonne 3, lignes 36-47; colonne 4, lignes 8-31; colonne 4, ligne 42 - colonne 5, ligne 40; colonne 10, lignes 18-68 *<br>--- | 1-4,6-9,11,13-15,17,25 | C 07 C 67/11<br>C 07 C 69/63<br>C 07 D 213/803<br>C 07 C 69/56<br>C 07 C 69/68<br>C 07 C 69/533 |
| Y | US-A-3 341 575 (W.L. FIERCE)<br>* Colonne 1, ligne 55 - colonne 2, ligne 28; colonne 4, ligne 61 - colonne 6, ligne 3; colonne 6, lignes 55-67 *<br>--- | 1-4,6-9,11,13-15,17,24,25 | |
| Y | FR-A-2 413 348 (DYNAMIT NOBEL)<br>* Page 5, lignes 12-18; page 7, lignes 8-24; page 8, lignes 10-28; page 9, lignes 17-38; page 10, lignes 7-20 *<br>----- | 1,8,9,10-15,17,18-21,23 | |

**DOMAINES TECHNIQUES RECHERCHES (Int. Cl.5)**

C 07 C 67/00
C 07 C 69/00

Le présent rapport a été établi pour toutes les revendications

| Lieu de la recherche | Date d'achèvement de la recherche | Examinateur |
|---|---|---|
| LA HAYE | 12-09-1991 | KINZINGER J.M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)